# EUROPEAN PATENT SPECIFICATION

(11) **EP 0 358 316 B1**
(45) Date of publication and mention of the grant of the patent: **27.10.1993**
(21) Application number: 89307305.6
(22) Date of filing: 19.07.1989
(51) Int. Cl.: A61F 5/441, B01D 53/02

(54) **Ostomy bag and gas filter for same**
Kolotomiebeutel und Gasfilter dafür
Poche d'ostomie et filtre à gaz pour son utilisation

(30) Priority: 15.08.1988 GB 8819384
(43) Date of publication of application: 14.03.1990
(73) Proprietor: E.R. Squibb & Sons, Inc., Princeton, N.J. 08540-4000 (US)
(72) Inventor: Plass, Ronald Andrew, Lindfield Sussex (GB); Steer, Graham Emery, Fulham London SW5 6A (GB)
(74) Representative: Cook, Anthony John

(56) References cited:
- EP-A- 0 210 032
- DE-A- 2 634 642
- FR-A- 2 310 739
- GB-A- 2 059 797

## Description

This invention relates to a gas filter arrangement in an ostomy bag, and to a method of assembling an ostomy bag including a filter.

There have been many attempts to include a gas vent and filter in an ostomy bag. Examples are described and illustrated in U.K. Patents Nos. 1,117,204; 1,379,464; 1,405,032; 1,462,492; 2,031,282; 2,083,760; 2,122,090; 2,150,029; 2,171,052; and 2,177,926.

U.K. Patent No. 2,059,797 lays stress on the desirability of avoiding gas by-passing the filter assembly. The construction illustrated in this patent involves provision of two separate heat seals (26, 27 in the patent Figure 2) and a further heat seal operation (28) to fix the filter assembly to the bag wall. Under present day conditions it is highly desirable to simplify design and manufacture, and the present invention was reached with this aim in mind. It has, moreover, been surprisingly found that a filter which tolerates some gas by-pass is nevertheless effective and useful in filtering and deodorising gases from an ostomy bag.

German Published Patent Application No. (DE-A)2634642 (McDonnell) shows an appliance for insertion in the anus. It has a filter pad loaded with activated carbon within a filter case. A film holds the pad within the case. The filter case is attached by screw threading to an annular holder and by that holder to a tubular and absorbent part of the appliance which is inserted into the stoma. This appliance does not address the basic problem of providing a filter suitable for attachment to an ostomy bag, nor the more specific problem of attaching filters to ostomy bags in a satisfactory manner during high-speed assembly.

According to the present invention there is provided a gas filter arrangement comprising a filter pad loaded with activated carbon, a moulded filter case, and a layer of plastics film attached to one surface of the pad so as to maintain the pad captive within the filter case characterised firstly in that the filter pad comprises open cell polyurethane foam loaded with the activated carbon having microfine non-woven gas permeable layers of plastics material on the two opposed surfaces of the pad, and secondly in that the filter case comprises a rim portion and an inwardly extending annular flanged portion, the layer of plastics film being microperforated and being attached to the surface of the rim portion remote from the flanged portion so as to maintain the filter pad within the rim portion and between the said layer of plastics film and the flanged portion.

Such a filter arrangement is preferably integrated into an ostomy bag by attaching a wall of the bag firstly to a region of the rim surface surrounding the flange by a heat seal or heat weld operation and secondly by attaching the bag wall to a film or layer on the filter pad, by means of a hot-melt adhesive matrix. At an appropriate location in the bag wall, a hole or slit (or several holes or slits) is provided to allow gas to escape to the ambient atmosphere. Though the words "firstly" or "secondly" have been used above, in the most preferred method of making a product according to this invention, the two aforesaid attachments are made substantially simultaneously in a single operation using a single hot platen.

An advantage of this procedure is that the filter arrangements can be stored in a magazine and can be fed to the appropriate location in a high speed machine. By a high speed machine is meant a machine capable of welding two sheets of film around their edges to produce an ostomy bag at a rate of at least 2000 and preferably 4000 bags per hour.

The invention will be better understood from the following description of one example of filter arrangement and bag according to the invention, given with reference to the accompanying illustrative drawings in which:-
Figure 1 is cross-sectional view of one example of filter disc according to the invention;
Figure 2 is a vertical cross-section taken in an axial plane through the centre of the filter and showing the upper portion of an ostomy bag; and
Figure 3 is an exploded view showing the construction and manner of assembly of the falter arrangement with one of the bag walls.

In the drawings, like parts are represented by like reference numerals.

A preferred example of filter arrangement according to the invention includes three basic parts, namely a moulded filter case 10 (Figure 3), a filter pad or disc 12, and a microperforated weldable film 14.

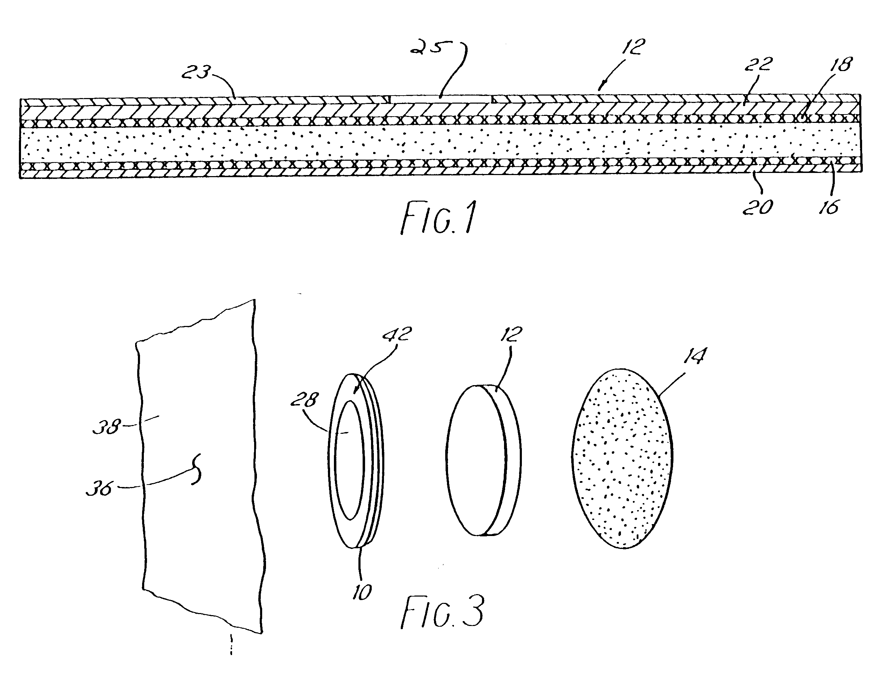

The construction of the filter pad 12 is shown in more detail in Figure 1. The preferred example of filter pad is formed by a 2 millimetre thick body of carbon-impregnated crushed polyurethane foam. The preferred shape for the filter pad is a thin disc but clearly is not essential to the invention that it should be circular nor of the specified thickness. Attached to the upper and lower surfaces of the disc by a matrix of adhesive known as "M-web adhesive" indicated by reference numerals 16 and 18 are a lower layer 20 of non-woven synthetic plastics material which is gas permeable and an upper layer 22 of microfine non-woven synthetic plastics material which is also gas permeable. One example of a suitable material for the layer 20 is that known as V115/463 supplied by Freudenberg U.K. and one example of a material suitable for the layer 22 is that known under the Trade Mark "Lutrovil 708" also available from Freudenberg U.K. Layers 20, 22 are permeable to the passage of gas through the layer parallel to their surfaces as well as normal to their surfaces.

The molded filter case 10 is preferably an annular piece of synthetic plastics material, e.g. ethylene vinyl acetate polymer, having a rim portion 24 and a flange portion 26. The flange 26 surrounds a hole 28. The internal diameter of the rim 24 is equal to or slightly greater than the external diameter of the filter pad 12, so that the filter pad fits snugly within the molded case 10. It is held therein by a microperforated weldable film 14 which is secured to the rear surface 30 of the rim 24 in any suitable manner, for example by heat welding or adhesive. The film 14 is, as stated, microperforated, and readily permits entry of gas from the interior 32 of the bag to the filter pad 12.

The filter arrangement so produced, consisting of the case 10, the pad 12 and the film 14, can readily be stored in a magazine and fed therefrom by suitable automatic means to a high speed production machine. In the preferred manner of manufacture for bags according to the invention, the filter arrangements are fed with the face of the flange 26 downwardly onto a sheet of bag film 38 (the presently preferred film being that known as MF bag film obtainable from W.R. Grace). The filter arrangements are fed so that the filter arrangement is substantially centralized over a cut or hole 36 in the bag film 38. In the illustrated embodiment, an S-shaped cut is shown but any other slit or hole or pattern of apertures allowing escape of gas would be suitable.

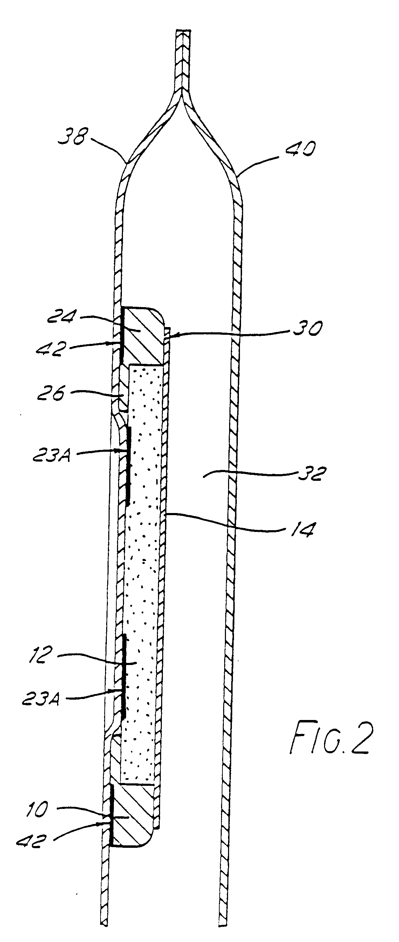

By application of a hot platen to the outer surface of the bag wall 38, the wall 38 is welded to the surface 42 (FIGS. 2 and 3) of the case 10 and due to the presence of the matrix of heat activated hot melt adhesive 23 on the surface of the layer 22, an annular region of this layer is adhesively secured to the rear surface of the bag wall 38. (See 23A, FIG. 2) In a central region of the layer 22, formed by annular region 23A, no adhesive join with the bag wall 38 is made in the area of the cut 36. For example, this area may be substantially one quarter of an inch (about 6 mm) in diameter. The lack of adhesive connection may be achieved, for example, by providing an appropriately located recess in the working surface of the hot platen, or, as illustrated in FIG. 1, by leaving the central region 25 without an adhesive layer.

As is conventional, a stomal orifice and a suitable means for securing the ostomy bag to the patient will be provided in one or other of the walls 38, 40 and, if desired, a bottom outlet opening to the bag can also be provided.

One advantage of the present invention is that the number of heat welding operations is reduced and hence manufacture is simplified, and, in addition, high speed manufacture can be employed in that filter arrangements according to the invention are particularly suited for rapid and automatic attachment to a film constituting a bag wall. Although no special measures are taken to constrain the path of the gas through the filtering arrangement, satisfactory filter and deodorizing efficiency is obtained. In addition, the filter is light and overall thickness of the filter is small which means that it is not obtrusive when worn under light clothing.

## Claims

1. A gas filter arrangement comprising a filter pad (12) loaded with activated carbon, a moulded filter case (10), and a layer of plastics film (14) attached to one surface of the pad (12) so as to maintain the pad (12) captive within the filter case characterised firstly in that the filter pad comprises open cell polyurethane foam loaded with the activated carbon having microfine non-woven gas permeable layers of plastics material (20,22) on the two opposed surfaces of the pad, and secondly in that the filter case (10) comprises a rim portion (24) and an inwardly extending annular flanged portion (26), the layer of plastics film (14) being microperforated and being attached to the surface of the rim portion (24) remote from the flanged portion (26) so as to maintain the filter pad within the rim portion (24) and between the said layer of plastics film (14) and the flanged portion (26).

2. An ostomy bag including a filter arrangement according to claim 1, the filter arrangement being integrated into the ostomy bag.

3. A method of assembling a combination comprising a gas filter arrangement according to claim 1 and a wall of a conventional ostomy bag, characterised by the steps of:
(a) attaching a wall (38) of the bag to a region (42) of the rim surface surrounding the flange (26) by a heat seal or a heat weld operation, and
(b) attaching the bag wall (38) to the said layer (22) on the filter pad by means of a hot-melt adhesive matrix (23).

4. A method according to claim 3 characterised in that the two aforesaid attachments are made substantially simultaneously in a single operation using a single hot platen.

## Patentansprüche

1. Gasfilteranordnung mit einem mit Aktivkohle beladenen Filterkissen (12), einem geformten Filtergehäuse (10) und einer Schicht aus Kunststoffolie (14), die derart an einer Oberfläche des Kissens (12) befestigt ist, daß das Kissen (12) innerhalb des Filtergehäuses gefangen ist, gekennzeichnet erstens dadurch, daß das Filterkissen mit der Aktivkohle beladenen offenzelligen Polyurethanschaum und mikrofeine, nichtgewebte gasdurchlässige Schichten aus Kunststoffmaterial (20, 22) auf den beiden gegenüberliegenden Oberflächen des Kissens aufweist, und zweitens dadurch, daß das Filtergehäuse (10) einen Randabschnitt (24) und einen sich nach innen erstreckenden Flanschabschnitt (26) aufweist, wobei die Schicht aus Kunststoffolie (14) mikroperforiert und an der dem Flanschabschnitt (26) abgewandten Oberfläche des Randabschnitts (24) befestigt ist, um das Filterkissen innerhalb des Randabschnitts (24) und zwischen der Schicht aus Kunststoffolie (14) und dem Flanschabschnitt (26) zu halten.

2. Kolotomiebeutel mit einer Filteranordnung gemäß Anspruch 1, wobei die Filteranordnung in den Kolotomiebeutel integriert ist.

3. Verfahren zum Zusammensetzen einer Kombination aus einer Gasfilteranordnung gemäß Anspruch 1 und einer Wand eines herkömmlichen Kolotomiebeutels, gekennzeichnet durch die folgenden Verfahrensschritte:
a) Befestigen einer Wand (38) des Beutels an einem Bereich (42) der den Flansch (26) umgebenden Randfläche mittels Heißsiegeln oder Heißschweißen, und
b) Befestigen der Beutelwand (38) an der Schicht (22) auf dem Filterkissen mittels einer Hot-Melt-Klebermatrix (23).

4. Verfahren gemäß Anspruch 3, dadurch gekennzeichnet, daß die beiden genannten Befestigungen im wesentlichen gleichzeitig in einem einzigen Vorgang unter Verwendung einer einzigen heißen Platte durchgeführt werden.

## Revendications

1. Dispositif de filtre à gaz comprenant un tampon filtrant (12) garni de charbon actif, une pièce moulée (10) formant logement de filtre, et une couche de pellicule (14) de matière plastique fixée à l'une des surfaces du tampon (12) de manière à maintenir le tampon (12) emprisonné à l'intérieur du logement de filtre, caractérisé premièrement en ce que le tampon filtrant comprend une mousse de polyuréthane à cellules ouvertes garnie du charbon actif, ayant des couches de matière plastique (20, 22) non tissée et microfine, perméables aux gaz, sur les deux surfaces opposées du tampon, et deuxièmement en ce que 1e logement de filtre (10) comprend un rebord (24) et une partie à bride (26) annulaire dirigée vers l'intérieur, la couche de pellicule (14) de matière plastique étant microperforée et étant fixée à la surface du rebord (24) éloignée de la partie à bride (26) de manière à maintenir le tampon filtrant à l'intérieur du rebord (24) et entre ladite couche de pellicule (14) de matière plastique et la partie à bride (26).

2. Poche de stomie comportant un dispositif de filtre selon la revendication 1, ce dispositif de filtre étant intégré dans la poche de stomie.

3. Procédé d'assemblage d'une combinaison comprenant un dispositif de filtre à gaz selon la revendication 1 et une paroi d'une poche de stomie classique, caractérisé par les opérations suivantes:
(a) fixation d'une paroi (38) de la poche à une région (42) de la surface du rebord entourant la bride (26), par un joint thermique ou par une opération de thermosoudage, et
(b) fixation de la paroi (38) de la poche à ladite couche (22) sur le tampon filtrant au moyen d'une matrice d'adhésif thermofusible (23)

4. Procédé selon la revendication 3, caractérisé en ce que les deux fixations précitées sont réalisées sensiblement en même temps et en une seule opération à l'aide d'un seul plateau chaud.
